(19) Europäisches Patentamt
European Patent Office
Office européen des brevets

(11) Publication number: **0 262 384 B1**

(12) **EUROPEAN PATENT SPECIFICATION**

(45) Date of publication of patent specification: **04.11.92**

(51) Int. Cl.[5]: **C07D 493/22**, A61K 31/365, A23K 1/17, A01N 43/90, //(C07D493/22,313:00,313:00, 311:00,307:00)

(21) Application number: **87112281.8**

(22) Date of filing: **25.08.87**

The file contains technical information submitted after the application was filed and not included in this specification

(54) **23-Deoxy derivatives of LL-F28249 Compounds.**

(30) Priority: **12.09.86 US 907186**

(43) Date of publication of application:
**06.04.88 Bulletin 88/14**

(45) Publication of the grant of the patent:
**04.11.92 Bulletin 92/45**

(84) Designated Contracting States:
**AT BE CH DE ES FR GB GR IT LI LU NL SE**

(56) References cited:
**EP-A- 0 002 615**
**EP-A- 0 214 731**
**EP-A- 0 254 583**
**US-A- 3 950 360**
**EP-A- 0 170 006**
**EP-A- 0 215 654**
**FR-A- 2 570 390**

(73) Proprietor: **AMERICAN CYANAMID COMPANY**
**1937 West Main Street P.O. Box 60**
**Stamford Connecticut 06904-0060(US)**

(72) Inventor: **Asato, Goro**
**10 Maddock Road**
**Titusville, NJ 08560(US)**
Inventor: **Tamura, Susan Yoshiko**
**35-02 Fox Run Drive**
**Plainsboro, NJ 08536(US)**

(74) Representative: **Wächtershäuser, Günter, Dr.**
**Tal 29**
**W-8000 München 2(DE)**

EP 0 262 384 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

The present invention relates to new derivatives of the antibiotics collectively defined as LL-F28249. These antibiotics preferably are produced by the fermentation of the microorganism Streptomyces cyaneogriseus subsp. noncyanogenus, deposited in NRRL under deposit accession no. 15773.

The present invention further relates to methods and compositions for preventing, treating or controlling helminths, ectoparasites, insects, acarids and nematodes infections or infestations in warm-blooded animals and agricultural crops by administering thereto prophylactically, therapeutically or pharmaceutically effective amount of the present 23-deoxy LL-F28249 agents (compounds), mixtures thereof or the pharmaceutically and pharmacologically-acceptable salts thereof.

These infections not only cause devastating effects to animals but also seriously effect the economics of farmers in raising meat-producing animals such as swine, sheep, cattle, goats, rabbits and poultry. Further, such infections are a source of great concern for companion animals such as horses, dogs and cats. Therefore, effective methods for the treatment and prevention of these diseases constantly are being sought.

### SUMMARY OF THE INVENTION

The present invention provides novel 23-deoxy derivatives of the compounds designated LL-F28249 (disclosed in EP-A-0 170 006) and represented by the following structural formula,

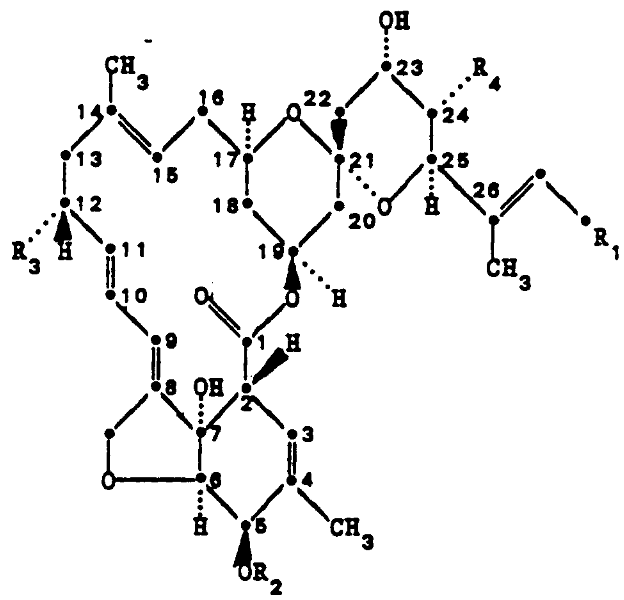

| Component | $R_1$ | $R_2$ | $R_3$ | $R_4$ |
|---|---|---|---|---|
| LL-F28249α | $CH(CH_3)_2$ | H | $CH_3$ | $CH_3$ |
| LL-F28249β | $CH_3$ | H | $CH_3$ | $CH_3$ |
| LL-F28249γ | $CH_3$ | $CH_3$ | $CH_3$ | $CH_3$ |
| LL-F28249ε | $CH(CH_3)_2$ | H | H | $CH_3$ |
| LL-F28249δ | $CH_2CH_3$ | H | $CH_3$ | $CH_3$ |
| LL-F28249θ | $CH(CH_3)_2$ | H | $CH_3$ | $CH_2CH_3$ |
| LL-F28249ι | $CH(CH_3)_2$ | H | $CH_2CH_3$ | $CH_3$ |
| LL-F28249λ | $CH(CH_3)_2$ | $CH_3$ | $CH_3$ | $CH_3$ |

The compounds of the present invention are represented by structural formula (I),

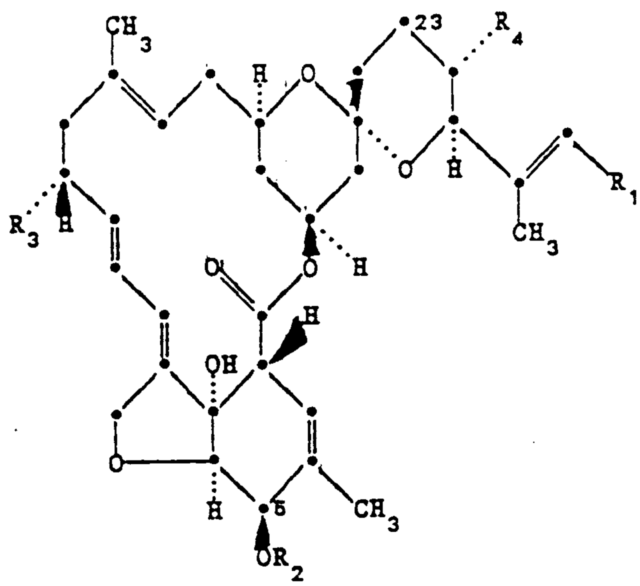

(I)

wherein $R_1$ is methyl, ethyl or isopropyl; $R_2$ is hydrogen or methyl; $R_3$ is hydrogen, methyl or ethyl; $R_4$ is methyl or ethyl; with the proviso that when $R_1$, $R_3$ and $R_4$ are methyl, $R_2$ cannot be hydrogen; and the pharmaceutically and pharmacologically acceptable salts thereof.

The compounds of the present invention are useful anthelmintics, ectoparasiticides, insecticides, acaricides and nematicides in treating, preventing or controlling such diseases in warm-blooded animals, such as poultry, cattle, sheep, swine, rabbits, horses, dogs, cats and human beings and in agricultural crops.

Although these diseases have been recognized for years and therapies exist for the treatment and prevention of the diseases, the present invention provides novel compounds in the search for effective such therapy. {Non pre-published documents EP-A-214 731, EP-A-215 654 and EP-A-254 583 disclose antibiotic substances of the milbemycin type.}

U.S. Patent 3,950,360, Aoki et al, April 13, 1976, discloses certain antibiotic substances obtained by culturing a Streptomyces microorganism, said compounds being useful as insecticides and acaricides. Further, an entire series of U.S. patents relates to certain compounds produced by the fermentation of Streptomyces avermitilis (U.S. Patent 4,171,314, Chabala et al, October 16, 1979; U.S. Patent 4,199,569, Chabala et al, April 22, 1980; U.S. Patent 4,206,205, Mrozik et al, June 3, 1980; U.S. Patent 4,310,519, Albers-Schonberg, January 12, 1982; U.S. Patent 4,333,925, Buhs et al, June 8, 1982). U.S. Patent 4,423,209, Mrozik, December 27, 1983 relates to the process of converting some of these less desirable components to more preferred ones. British Patent Application 2166436A of Ward et al relates to antibiotics also.

The present compounds or the pharmaceutically and pharmacologically-acceptable salts thereof exhibit excellent and effective treatment and/or prevention of these serious diseases of warm-blooded animals.

It is an object of the present invention, therefore, to provide novel 23-deoxy compounds of the LL-F28249 series of compounds.

It is a further object of the present invention to provide novel compositions or methods for the treatment, prevention or control of helminth, ectoparasite, insect, acarid and nematode infections and infestations in agricultural crops.

It also is an object of the present invention to provide novel compositions for use to effectively control, prevent or treat said diseases in warm-blooded animals.

These and further objects will become apparent by the below-provided detailed description of the invention.

DETAILED DESCRIPTION OF THE INVENTION

The compounds of the invention are represented by structural formula (I),

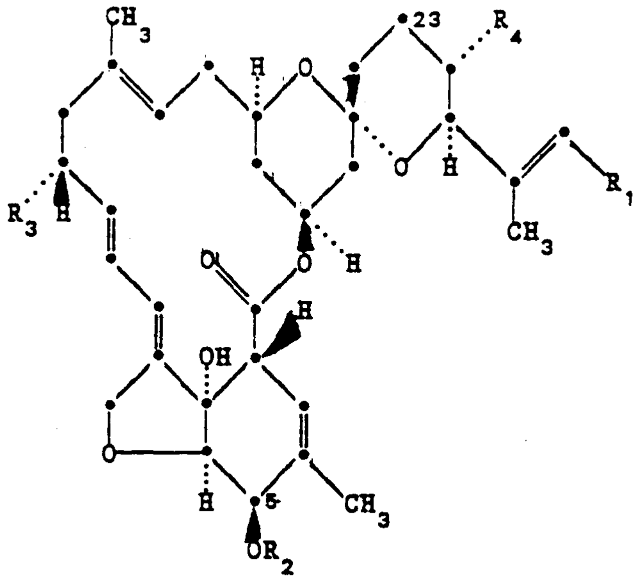

(I)

wherein $R_1$ is methyl, ethyl or isopropyl; $R_2$ is hydrogen or methyl; $R_3$ is hydrogen, methyl or ethyl; $R_4$ is methyl or ethyl; with the proviso of claim 1 and the pharmaceutically and pharmacologically acceptable salts thereof.

Preferably, $R_1$ is isopropyl; $R_2$ is hydrogen or methyl; $R_3$ is methyl; and $R_4$ is methyl. Most preferred compound includes $R_1$ as isopropyl, $R_2$ as hydrogen, $R_3$ as methyl and $R_4$ as methyl.

The 23-deoxy derivatives of LL-F28249 are prepared by converting the 23-hydroxyl group to a 23-halo compound. This is then reduced with a reducing agent such as tributyltin hydride in the presence of a free radical initiator such as azobisisobutyronitrile to afford the 23-deoxy-LL-F28249 compound.

The preferred 23-halo compound is 23-bromo-LL-F28249 compound which is readily prepared by reacting the appropriate LL-F28249 compound with about 1.0 to 1.5 molar equivalents of triphenylphosphine dibromide, preferably 1.0 to 1.25 molar equivalents, in an inert solvent such as acetonitrile, carbon tetrachloride, dimethylformamide or benzonitrile under $N_2$ atmosphere at -20 ° C to 25 ° C, preferably 0 ° C to 25 ° C. The reaction occurs selectively at the 23 position. The bromide is then reduced with a reducing agent such as tributyltin hydride in the presence of a free radical initiator such as azobisisobutyronitrile in an inert solvent such as toluene or xylene at reflux temperature to give the 23-deoxy-LL-F28249 compound.

The compounds of the present invention are useful as anthelmintics, ectoparasiticides, insecticides, acaricides and nematicides.

A preferred method of controlling plant insects in accordance with the present invention is defined in claims 3 and 4.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Parascaris. Certain of these, such as Nematodirus, Cooperia, and Oesophagostomum primarily attack the intestinal tract, while others, such as Haemonchus and Ostertagia, are most prevalent in the stomach. Still others, such as Dictyocaulus, are found in the lungs. However, other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs, and, if left untreated, may result in

death of the infected host. The LL-F28249 compound derivatives of the present invention unexpectedly have high activity against these parasites. Additionally, the compounds of this invention also are active against Drofilaria in dogs, Nematospiroides, Syphacia, Aspiculuris in rodents, arthropod ectoparasites such as ticks, mites, lice, fleas, blowfly, of animals and birds, the ectopara site Lucilia sp. of sheep, biting insects and migrating dipterous larvae such as Hypoderma sp. in cattle, Gastrophilus in horses and Cuterebra sp. in rodents.

The compounds of the present invention also are useful in treating, preventing or controlling parasites (collectively includes ecto and/or endoparasites) which infect human beings, as well. The most common genera of parasites of the gastrointestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra-intestinal stages of the intestinal worms Strongyloides and Trichinella. The present compounds also are of value against arthropods parasitizing man, biting insects and other dipterous pests causing annoyance to man.

These compounds further are active against household pests such as the cockroach, Blattella sp., clothes moth, Tineola sp., carpet beetle Attagenus sp. and the housefly Musca domestica.

Insect pests of stored grains such as Tribolium sp., Tenebrio sp., and of agricultural plants such as spider mites (Tetranychus sp.), aphids (Acyrthiosiphon sp.), southern army worms, tobacco budworms, boll weevils migratory orthopterans, such as locusts and immature stages of insects living on plant tissue are controlled by the present compounds, as well as the control of soil nematodes and plant parasites such as Meloidogyne sp.

The compounds of the present invention may be administered orally or parenterally for animal and human usage, while they may be formulated in liquid or solid form for agricultural use. Oral administrations may take the form of a unit dosage form such as a capsule, bolus or tablet, or as a liquid drench where used as an anthelmintic for animals.

The animal drench is normally a solution, suspension or dispersion of the active compound, usually in water, together with a suspending agent such as bentonite and a wetting agent or like excipient. Generally, the drenches also contain an antifoaming agent. Drench formulations generally contain about 0.00l% to 0.5%, by weight, of the active compound. Preferred drench formulations contain about 0.01% to 0.1% by weight.

Capsules and boluses comprise the active ingredient admixed with a carrier vehicle such as starch, talc, magnesium stearate or di-calcium phosphate.

Where it is desired to administer the 23-deoxy LL-F28249 derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the active compound depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the active compounds of the invention may be administered to animals parenterally such as by intraruminal, intramuscular, intratracheal or subcutaneous injection. In such an event, the active compound is dissolved or dispersed in a liquid carrier vehicle.

For parenteral administration, the active compound is suitably admixed with an acceptable vehicle, preferably a vegetable oil such as peanut oil, cotton seed oil or the like. Other parenteral vehicles such as org anic preparations using solketal, glycerol formal and aqueous parenteral formulation also are used. The active LL-F28249 compound derivative or derivatives are dissolved or suspended in the parenteral formulation for administration. Such formulations generally contain about 0.005% to 5%, by weight, of the active compound.

Although the compounds of the present invention are primarily used in the treatment, prevention or control of helminthiasis, they also are useful in the prevention, treatment or control of diseases caused by other parasites (collectively both ecto and/or endoparasites). For example, arthropod parasites such as ticks, lice, fleas, mites and other biting insects in domesticated animals and poultry are controlled by the present compounds. These compounds also are effective in treatment of parasitic diseases which occur in other animals including human beings. The optimum amount to be employed will, of course, depend upon the particular compound employed, the species of animal to be treated and the type and severity of parasitic infection or infestation. Generally, the amount useful in oral administration of these novel compounds is

about 0.001 mg to 10 mg per kg of animal body weight, such total dose being given at one time or in divided doses over a relatively short period of time (1-5 days). The preferred compounds of the invention give excellent control of such parasites in animals by administering about 0.025 mg to 3 mg per kg of animal body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of animals' feed or dissolved or suspended in the drinking water, compositions are provided in which the active compound or compounds are intimately dispersed in an inert carrier or diluent. An inert carrier is one that will not react with the active component and that will be administered safely to animals. Preferably, a carrier for feed administration is one that is, or may be, an ingredient of the animal ration.

Suitable compositions include feed premixes or supplements in which the active compound is present in relatively large amounts wherein said feed premixes or supplements are suitable for direct feeding to the animal or for addition to the feed either directly or after an intermediate dilution or blending step.

Typical carriers or diluents suitable for such compositions include distillers' dried grains, corn meal, citrus meal, fermentation residues, ground oyster shells, wheat shorts, molasses solubles, corn cob meal, edible bean mill feed, soya grits, crushed limestone and the like. The active compounds are intimately dispersed throughout the carrier by methods such as grinding, stirring, milling or tumbling. Compositions containing about 0.005% to 2.0%, by weight, of the active compound are particularly suitable as feed premixes.

Feed supplements, which are fed directly to the animal, contain about 0.0002% to 0.3%, by weight, of the active compounds. Such supplements are added to the animal feed in an amount to give the finished feed the concentration of active compound desired for the treatment and control of parasitic diseases. Although the desired concentration of active compound will vary depending upon the factors previously mentioned as well as upon the particular derivative employed, the compounds of this invention are usually fed at concentrations of about 0.00001% to 0.02% in the feed in order to achieve the desired antiparasitic result.

The compounds of this invention also are useful in combating agricultural pests that inflict damage upon growing or stored crops. The present compounds are applied, using known techniques such as sprays, dusts, emulsions and the like, to the growin g or stored crops to effect protection from agricultural pests, or to the foliage of crops and plants, the soil in which they are grown at the trunk thereof

The present invention is illustrated by the following examples which are illustrative of said invention and not limitative thereof.

EXAMPLE 1

23-Bromo-LL-F28249α

In 10 mL of dry acetonitrile, 0.161 g of triphenylphosphine and 28 μL of bromine are dissolved, and this solution is added dropwise under $N_2$ atmosphere to 262.2 mg of LL-F28249α in 5 mL of dry acetonitrile cooled in an ice bath. After stirring for 45 minutes in the ice bath, the reaction mixture is stirred at room temperature for 19 hours. The reaction is quenched with 10 drops of water. The mixture is evaporated to dryness, and the residue is chromatographed on silica gel using 0.75-1.0% i-PrOH in $CH_2Cl_2$. After stripping of solvents, this yields 75.7 mg of the title compound which is identified by mass spectrometry and NMR spectroscopy.

EXAMPLE 2

23-Deoxy-LL-F28249α

In 2 mL of toluene, 35.9 mg of 23-bromo-LL-F28249α, a catalytic amount of azobisisobutyronitrile (AIBN) and 17 μL of tributyltin hydride under $N_2$ atmosphere are heated at reflux temperature for 0.5 hours. The mixture is evaporated to dryness, and the residue is chromatographed on silica gel using initially $CH_2Cl_2$ and then 1% i-PrOH in $CH_2Cl_2$ as eluents. The fractions from the latter solvent mixture contain the product with traces of tin compounds. Partitioning between acetonitrile and hexane affords separation of the desired product in the acetonitrile layer. Further extraction of the hexane layer with acetonitrile affords additional product. The combined acetonitrile layers are evaporated to dryness. Again, the product is characterized by mass spectrometry and NMR spectroscopy.

EXAMPLES 3 AND 4

23-Bromo-LL-F28249γ

Following the procedures of Example 1, LL-F28249γ is converted into the title compound and identified by mass spectrometry and NMR spectroscopy.

Similarly, LL-F28249λ is converted to 23-bromo-LL-F28249λ.

EXAMPLES 5 AND 6

23-Deoxy-LL-F28249γ

Using the procedure of Example 2, 23-bromo-LL-F28249γ is reduced with tributyltin hydride to give the title compound which is identified by mass spectrometry and NMR spectroscopy.

Similarly, 23-bromo-LL-F28249λ is reduced to afford 23-deoxy-LL-F28249λ.

EXAMPLES 7-11

Using the procedures described in Example 1 and 2, the following 23-deoxy-LL-F28249 compounds are prepared:

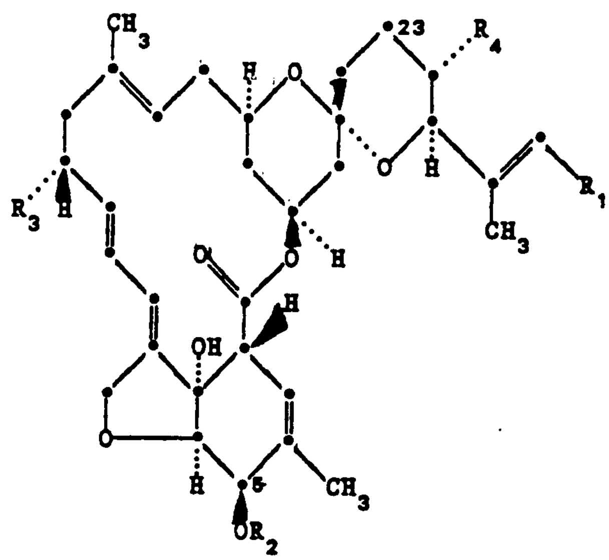

(I)

| $R_1$ | $R_2$ | $R_3$ |
|---|---|---|
| $CH_3$ | $CH_3$ | $CH_3$ |
| $CH_3(CH_3)_2$ | H | $CH_3$ |
| $CH_2CH_3$ | $CH_3$ | $CH_3$ |
| $CH(CH_3)_2$ | CH3 | $CH_2CH_3$ |
| $CH(CH_3)_2$ | $CH_2CH_3$ | $CH_3$ |

**Claims**

**Claims for the following Contracting States : BE, CH, DE, FR, GB, IT, LI, LU, NL, SE**

**1.** The compounds characterized by structural formula (I),

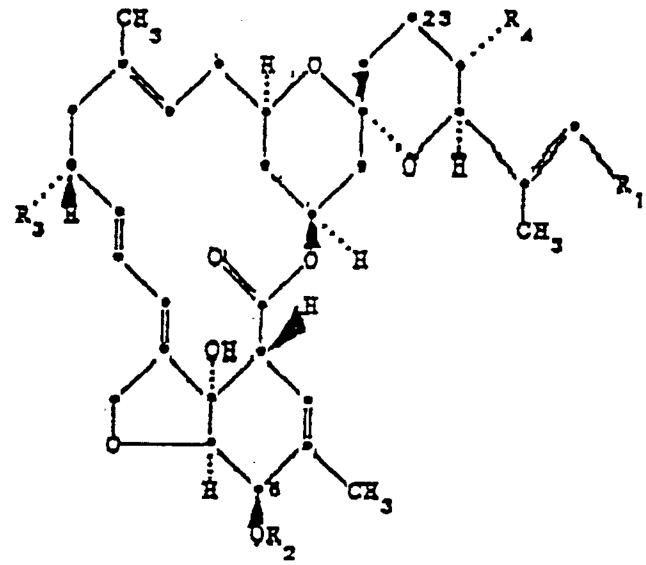

(I)

wherein $R_1$ is methyl, ethyl or isopropyl; $R_2$ is hydrogen or methyl; $R_3$ is hydrogen, methyl or ethyl; $R_4$ is methyl or ethyl; with the proviso that when $R_1$, $R_3$ and $R_4$ are methyl, $R_2$ cannot be hydrogen; and the pharmaceutically and pharmacologically acceptable salts thereof.

**2.** A compound according to Claim 1, wherein $R_1$ is isopropyl; $R_2$ is hydrogen or methyl; $R_3$ is methyl; and $R_4$ is methyl; and wherein $R_1$ is isopropyl; $R_2$ is hydrogen; $R_3$ is methyl; and $R_4$ is methyl.

**3.** A method for controlling plant insects, topically or systemically, and protecting crops, trees, shrubs, stored grain and ornamentals, said method characterized by: applying an insecticidally-effective amount of the compound represented by the structural formula (I) as defined in claim 1, and the pharmaceutically and pharmacologically acceptable salts thereof.

**4.** A method according to Claim 3, wherein said compound is applied to the foliage of crops and plants, the soil in which they are grown or the trunk thereof.

**5.** A method according to Claim 4, wherein said compound has $R_1$ as isopropyl; $R_2$ is hydrogen or methyl; $R_3$ is methyl; and $R_4$ is methyl; and wherein said compound is $R_1$ as isopropyl; $R_2$ is hydrogen; $R_3$ is methyl; and $R_4$ is methyl.

**6.** A method for the control of plant nematodes, said method characterized by: applying to the foliage of plants, the soil in which they are grown or into the trunks thereof, a nematocidally-effective amount of the compound represented by structural formula (I), as defined in claim 1, and the pharmaceutically and pharmacologically acceptable salts thereof.

**7.** A method according to Claim 6, wherein said compound has $R_1$ as isopropyl; $R_2$ is hydrogen or methyl; $R_3$ is methyl; and $R_4$ is methyl; and wherein said compound has $R_1$ as isopropyl; $R_2$ is hydrogen; $R_3$ is methyl; and $R_4$ is methyl.

**8.** A composition for the treatment, prevention or control of parasitic infections in warm-blooded animals, said composition characterized by: a prophylactically, therapeutically or pharmaceutically-effective amount of the compound represented by structural formula (I), as defined in claim 1, or the pharmaceutically and pharmacologically acceptable salts thereof; and an inert carrier.

**9.** A composition for controlling plant insects, said composition characterized by: an insecticidally-effective

amount of the compound represented by structural formula (I), as defined in claim 1, or the pharmaceutically and pharmacologically acceptable salts thereof; and an inert carrier.

**Claims for the following Contracting States : AT, ES, GR**

1. A process for preparing the compounds characterized by structural formula (I)

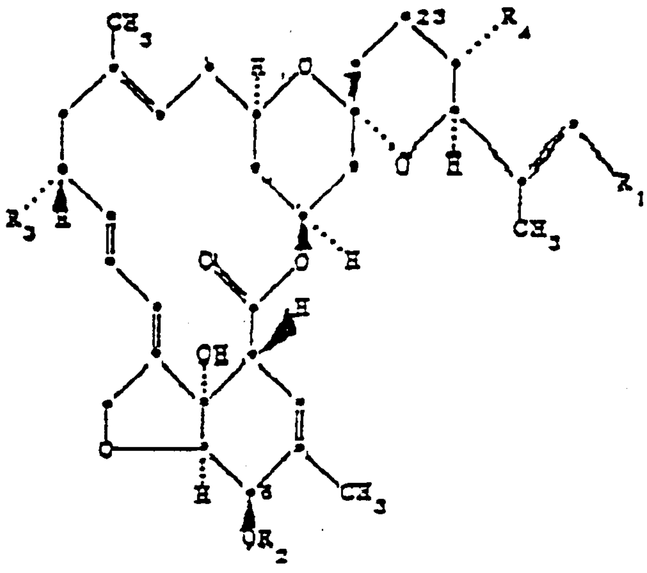

(I)

wherein $R_1$ is methyl, ethyl or isopropyl; $R_2$ is hydrogen or methyl; $R_3$ is hydrogen, methyl or ethyl; $R_4$ is methyl or ethyl; with the proviso that when $R_1$, $R_3$ and $R_4$ are methyl, $R_2$ cannot be hydrogen; and the pharmaceutically and pharmalogically acceptable salts thereof, which comprises reacting a LL-F28249 compound having a 23-hydroxyl group with about 1.0 to 1.5 molar equivalents of triphenyl phosphine dibromide in an inert solvent under $N_2$ atmosphere at -20°C to 25°C and reducing the obtained 23-bromo-LL-F28249 compound with a reducing agent in the presence of a free radical initiator in an inert solvent at reflux temperature to give the 23-deoxy-LL-F28249 compound (I).

2. A process according to Claim 1, wherein $R_1$ is isopropyl; $R_2$ is hydrogen or methyl; $R_3$ is methyl; and $R_4$ is methyl; and wherein $R_1$ is isopropyl; $R_2$ is hydrogen; $R_3$ is methyl; and $R_4$ is methyl.

3. A method for controlling plant insects, topically or systemically, and protecting crops, trees, shrubs, stored grain and ornamentals, said method characterized by: applying an insecticidally-effective amount of the compound represented by the structural formula (I) as defined in Claim 1, and the pharmaceutically and pharmalogically acceptable salts thereof.

4. A method according to Claim 3, wherein said compound is applied to the foliage of crops and plants, the soil in which they are grown or the trunk thereof.

5. A method according to Claim 4, wherein said compound has $R_1$ as isopropyl; $R_2$ is hydrogen or methyl; $R_3$ is methyl; and $R_4$ is methyl; and wherein said compound is $R_1$ as isopropyl; $R_2$ is hydrogen; $R_3$ is methyl; and $R_4$ is methyl.

6. A method for the control of plant nematodes, said method characterized by: applying to the foliage of plants, the soil in which they are grown or into the trunks thereof, a nematocidally-effective amount of the compound represented by structural formula (I), as defined in Claim 1, and the pharmaceutically and pharmalogically acceptable salts thereof.

7. A method according to Claim 6, wherein said compound has $R_1$ as isopropyl; $R_2$ is hydrogen or methyl; $R_3$ is methyl; and $R_4$ is methyl; and wherein said compound has $R_1$ as isopropyl; $R_2$ is

hydrogen; $R_3$ is methyl; and $R_4$ is methyl.

8. A composition for the treatment, prevention or control of parasitic infections in warm-blooded animals, said composition characterized by: a prophylactically, therapeutically or pharmaceutically-effective amount of the compound represented by structural formula (I), as defined in Claim 1, or the pharmaceutically and pharmacologically acceptable salts thereof; and an inert carrier.

9. A composition for controlling plant insects, said composition characterized by: an insecticidally-effective amount of the compound represented by structural formula (I), as defined in Claim 1, or the pharmaceutically and pharmacologically acceptable salts thereof; and an inert carrier.

**Patentansprüche**
**Patentansprüche für folgende Vertragsstaaten : BE, CH, DE, FR, GB, IT, LI, LU, NL, SE**

1. Die Verbindungen, die durch die Strukturformel (I) charakterisiert sind

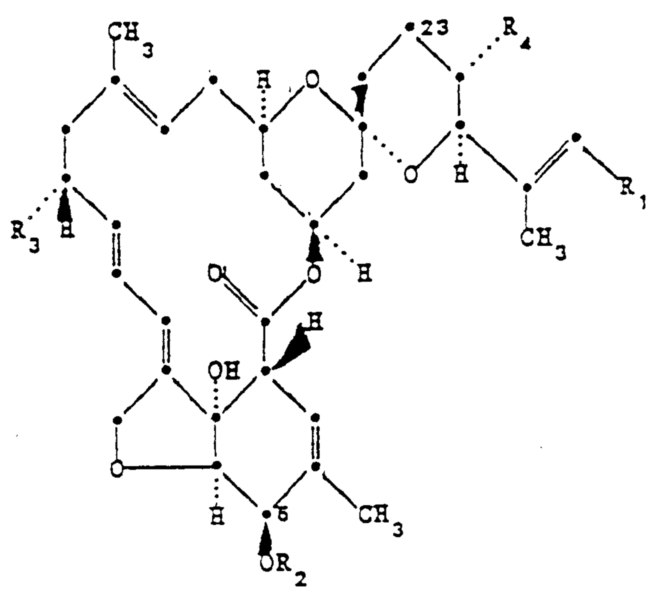

(I)

worin $R_1$ Methyl, Ethyl oder Isopropyl ist; $R_2$ Wasserstoff oder Methyl ist; $R_3$ Wasserstoff, Methyl oder Ethyl ist; $R_4$ Methyl oder Ethyl ist, mit der Bedingung, daß wenn $R_1$, $R_3$ und $R_4$ Methyl sind, $R_2$ nicht Wasserstoff sein kann; sowie deren pharmazeutisch und pharmakologisch akzeptable Salze.

2. Eine Verbindung nach Anspruch 1, worin $R_1$ Isopropyl ist; $R_2$ Wasserstoff oder Methyl ist; $R_3$ Methyl und $R_4$ Methyl ist und worin $R_1$ Isopropyl ist; $R_2$ Wasserstoff ist; $R_3$ Methyl und $R_4$ Methyl ist.

3. Ein Verfahren zum örtlichen oder systematischen Kontrollieren von Pflanzeninsekten und zum Schützen von Feldfrüchten, Bäumen, Sträuchern, gelagertem Getreide und Zierpflanzen, wobei das Verfahren charakterisiert ist durch: Aufbringen einer insektizid wirksamen Menge der Verbindung der Strukturformel (I), wie sie in Anspruch 1 definiert ist und deren pharmazeutisch und pharmakologisch akzeptable Salze.

4. Ein Verfahren nach Anspruch 3, worin die genannte Verbindung auf das Laubwerk der Feldfrüchte und Pflanzen, den Boden, in dem sie wachsen, oder deren Halme bzw. Stämme aufgebracht wird.

5. Ein Verfahren nach Anspruch 4, worin bei der genannten Verbindung $R_1$ Isopropyl ist; $R_2$ Wasserstoff oder Methyl ist; $R_3$ Methyl und $R_4$ Methyl ist und wobei in der genannten Verbindung $R_1$ Isopropyl ist; $R_2$ Wasserstoff ist; $R_3$ Methyl und $R_4$ Methyl ist.

6. Ein Verfahren zum Kontrollieren von Pflanzen-Nematoden, das charakterisiert ist durch: Aufbringen einer nematozid wirksamen Menge der Verbindung der Strukturformel (I), wie sie in Anspruch 1 definiert ist und deren pharmazeutisch und pharmakologisch akzeptable Salze, auf das Laubwerk von Pflanzen, den Boden in dem sie wachsen oder auf deren Halme bzw. Stämme.

7. Ein Verfahren nach Anspruch 6, worin bei der genannten Verbindung $R_1$ Isopropyl ist; $R_2$ Wasserstoff oder Methyl ist; $R_3$ Methyl und $R_4$ Methyl ist und wobei in der genannten Verbindung $R_1$ Isopropyl ist; $R_2$ Wasserstoff ist; $R_3$ Methyl und $R_4$ Methyl ist.

8. Zubereitung für die Behandlung, Verhinderung oder Kontrolle parasitärer Infektionen in warmblütigen Tieren, wobei die Zubereitung charakterisiert ist durch: eine prophylaktisch, therapeutisch oder pharmazeutisch wirksame Menge der Verbindung der Strukturformel (I), wie sie in Anspruch 1 definiert ist oder von deren pharmazeutisch und pharmakologisch akzeptablen Salzen und einen inerten Träger.

9. Eine Zubereitung zum Kontrollieren von Pflanzeninsekten, wobei die genannte Zubereitung charakterisiert ist durch: eine insektizid wirksame Menge der Verbindung der Strukturformel (I), wie sie in Anspruch 1 definiert ist oder deren pharmazeutisch oder pharmakologisch akzeptable Salze und einen inerten Träger.

**Patentansprüche für folgende Vertragsstaaten : AT, ES, GR**

1. Ein Verfahren zum Herstellen der Verbindungen, die durch die Strukturformel (I) charakterisiert sind

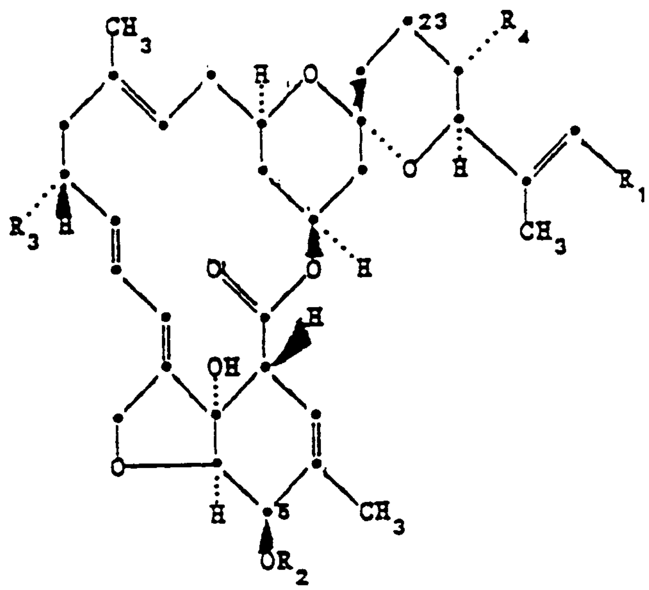

(I)

worin $R_1$ Methyl, Ethyl oder Isopropyl ist; $R_2$ Wasserstoff oder Methyl ist; $R_3$ Wasserstoff, Methyl oder Ethyl ist; $R_4$ Methyl oder Ethyl ist, mit der Bedingung, daß wenn $R_1$, $R_3$ und $R_4$ Methyl sind, $R_2$ nicht Wasserstoff sein kann; sowie deren pharmazeutisch und pharmakologisch akzeptable Salze, umfassend die Umsetzung einer LL-F28249-Verbindung, die eine 23-Hydroxylgruppe aufweist, mit etwa 1,0 bis 1,5 molaren Äquivalenten von Triphenylphosphindibromid in einem inerten Lösungsmittel unter $N_2$-Atmosphäre bei -20 ° C bis 25 ° C und Reduzieren der erhaltenen 23-Brom-LL-F28249-Verbindung mit einem Reduktionsmittel in Gegenwart eines Initiators freier Radikale in einem inerten Lösungsmittel bei Rückflußtemperatur zur 23-Desoxy-LL-F28249-Verbindung (I).

2. Ein Verfahren nach Anspruch 1, worin $R_1$ Isopropyl ist; $R_2$ Wasserstoff oder Methyl ist; $R_3$ Methyl und $R_4$ Methyl ist und worin $R_1$ Isopropyl ist; $R_2$ Wasserstoff ist; $R_3$ Methyl und $R_4$ Methyl ist.

3. Ein Verfahren zum örtlichen oder systematischen Kontrollieren von Pflanzeninsekten und zum Schützen

von Feldfrüchten, Bäumen, Sträuchern, gelagertem Getreide und Zierpflanzen, wobei das Verfahren charakterisiert ist durch: Aufbringen einer insektizid wirksamen Menge der Verbindung der Strukturformel (I), wie sie in Anspruch 1 definiert ist und deren pharmazeutisch und pharmakologisch akzeptable Salze.

**4.** Ein Verfahren nach Anspruch 3, worin die genannte Verbindung auf das Laubwerk der Feldfrüchte und Pflanzen, den Boden, in dem sie wachsen, oder deren Halme bzw. Stämme aufgebracht wird.

**5.** Ein Verfahren nach Anspruch 4, worin bei der genannten Verbindung $R_1$ Isopropyl ist; $R_2$ Wasserstoff oder Methyl ist; $R_3$ Methyl und $R_4$ Methyl ist und wobei in der genannten Verbindung $R_1$ Isopropyl ist; $R_2$ Wasserstoff ist; $R_3$ Methyl und $R_4$ Methyl ist.

**6.** Ein Verfahren zum Kontrollieren von Pflanzen-Nematoden, das charakterisiert ist durch: Aufbringen einer nematozid wirksamen Menge der Verbindung der Strukturformel (I), wie sie in Anspruch 1 definiert ist und deren pharmazeutisch und pharmakologisch akzeptable Salze, auf das Laubwerk von Pflanzen, den Boden in dem sie wachsen oder auf deren Halme bzw. Stämme.

**7.** Ein Verfahren nach Anspruch 6, worin bei der genannten Verbindung $R_1$ Isopropyl ist; $R_2$ Wasserstoff oder Methyl ist; $R_3$ Methyl und $R_4$ Methyl ist und wobei in der genannten Verbindung $R_1$ Isopropyl ist; $R_2$ Wasserstoff ist; $R_3$ Methyl und $R_4$ Methyl ist.

**8.** Eine Zubereitung für die Behandlung, Verhinderung oder Kontrolle parasitärer Infektionen in warmblütigen Tieren, wobei die Zubereitung charakterisiert ist durch: eine prophylaktisch, therapeutisch oder pharmazeutisch wirksame Menge der Verbindung der Strukturformel (I), wie sie in Anspruch 1 definiert ist oder von deren pharmazeutisch und pharmakologisch akzeptablen Salzen und einen inerten Träger.

**9.** Eine Zubereitung zum Kontrollieren von Pflanzeninsekten, wobei die genannte Zubereitung charakterisiert ist durch: eine insektizid wirksame Menge der Verbindung der Strukturformel (I), wie sie in Anspruch 1 definiert ist oder deren pharmazeutisch oder pharmakologisch akzeptable Salze und einen inerten Träger.

**Revendications**
**Revendications pour les Etats contractants suivants : BE, CH, DE, FR, GB, IT, LI, LU, NL, SE**

**1.** Composés caractérisés par la formule développée suivante :

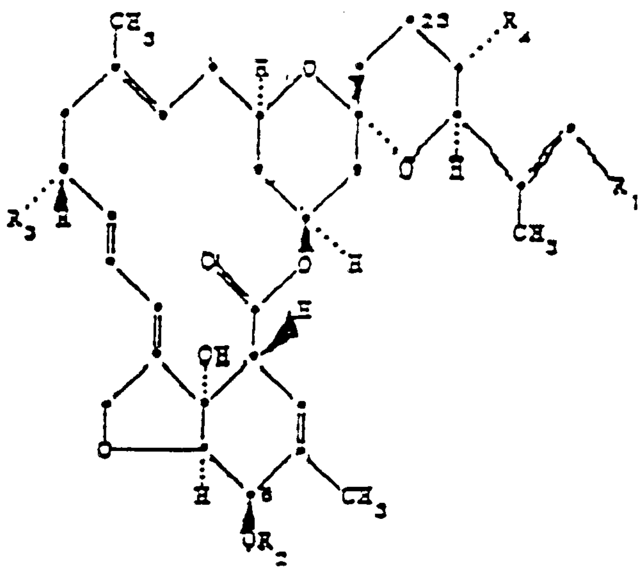

(I)

où $R_1$ est un méthyle, éthyle, ou isopropyle ; $R_2$ est un hydrogène ou un méthyle ; $R_3$ est un hydrogène, un méthyle ou un éthyle ; $R_4$ est un méthyle ou un éthyle, à la condition que quand $R_1$, $R_3$ et $R_4$ sont des méthyles, $R_2$ ne peut être un hydrogène ; et leurs sels acceptables aux points de vue pharmacologique et pharmaceutique.

**2.** Composé suivant la revendication 1, où $R_1$ est un isopropyle ; $R_2$ est un hydrogène ou un méthyle ; $R_3$ est un méthyle ; $R_4$ est un méthyle ; et où $R_1$ est un isopropyle ; $R_2$ est un hydrogène ; $R_3$ est un méthyle ; $R_4$ est un méthyle.

**3.** Procédé pour combattre les insectes des végétaux, de façon locale ou systémique, et pour protéger les plantes cultivées, les arbres, les arbustes, le grain stocké et les plantes ornementales, ledit procédé étant caractérisé par l'application d'une quantité efficace en tant qu'insecticide du composé représenté par la formule développée (I), comme définie dans la revendication 1, et leurs sels acceptables aux points de vue pharmacologique et pharmaceutique.

**4.** Procédé selon la revendication 3, dans lequel ledit composé est appliqué au feuillage des cultures et plantes, sur le sol de culture ou sur leur tronc.

**5.** Procédé selon la revendication 4, dans lequel ledit composé comprend $R_1$ qui est un isopropyle ; $R_2$ un hydrogène ou un méthyle ; $R_3$ un méthyle ; $R_4$ un méthyle ; et dans lequel ledit composé comprend $R_1$ qui est un isopropyle ; $R_2$ un hydrogène ; $R_3$ un méthyle ; $R_4$ un méthyle.

**6.** Procédé pour contrôler les nématodes des végétaux, ledit procédé étant caractérisé par l'application au feuillage des plantes, sur le sol de culture ou sur leur tronc d'une quantité efficace en tant que nématocide du composé représenté par la formule développée (I), comme définie dans la revendication 1, et leurs sels acceptables aux points de vue pharmacologique et pharmaceutique.

**7.** Procédé selon la revendication 6, dans lequel ledit composé comprend $R_1$ qui est un isopropyle ; $R_2$ un hydrogène ou un méthyle ; $R_3$ un méthyle et $R_4$ un méthyle ; et dans lequel ledit composé comprend $R_1$ qui est un isopropyle ; $R_2$ un hydrogène ; $R_3$ un méthyle et $R_4$ un méthyle.

**8.** Composition pour le traitement, la prévention ou le contrôle des infections parasitaires des animaux à sang chaud, ladite composition étant caractérisée par : une quantité efficace aux points de vue prophylactique, thérapeutique ou pharmaceutique du composé représenté par la formule développée

(I), ou leurs sels acceptables aux points de vue pharmacoloqique et pharmaceutique ; et un vecteur inerte.

9. Composition pour combattre les insectes des végétaux, ladite composition étant caractérisée par : une quantité efficace en tant qu'insecticide du composé représenté par la formule développée (I), ou leurs sels acceptables aux points de vue pharmacologique et pharmaceutique ; et un vecteur inerte.

**Revendications pour les Etats contractants suivants : AT, ES, GR**

1. Procédé pour préparer les composés caractérisés par la formule développée suivante :

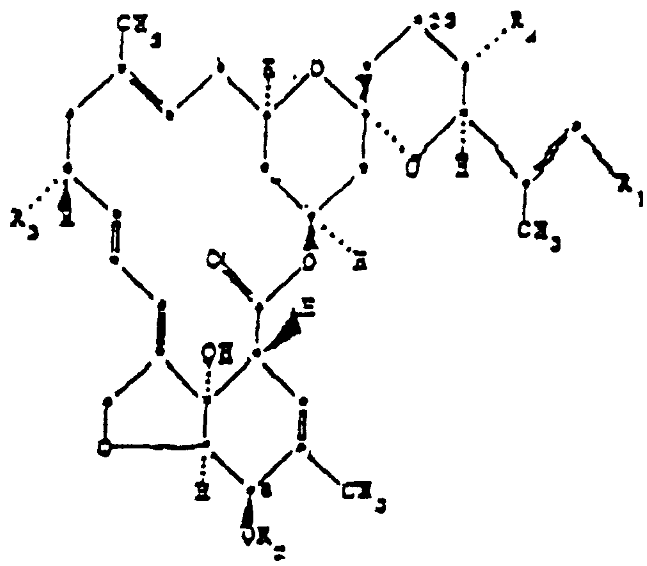

(I)

où $R_1$ est un méthyle, éthyle, ou isopropyle ; $R_2$ est un hydrogène ou un méthyle ; $R_3$ est un hydrogène, un méthyle ou un éthyle ; $R_4$ est un méthyle ou un éthyle, à la condition que quand $R_1$, $R_3$ et $R_4$ sont des méthyles, $R_2$ ne peut être un hydrogène ; et leurs sels acceptables aux points de vue pharmacologique et pharmacautique qui comprend la réaction du composé LL-F28249 ayant un groupement 23-hydroxyle avec environ 1,0 à 1,5 équivalents molaires de dibromure de triphénylphosphine, dans un solvant inerte sous atmosphère de $N_2$ entre -20 ° C et 25 ° C, et la réduction du composé 23-bromo-LL-28249 obtenu avec un agent réducteur en présence d'un initiateur de radicaux libres, dans un solvant inerte à la température de reflux, pour donner le composé 23-désoxy-LL-F28249.

2. Procédé suivant la revendication 1, où $R_1$ est un isopropyle ; $R_2$ est un hydrogène ou un méthyle ; $R_3$ est un méthyle ; $R_4$ est un méthyle ; et où $R_1$ est un isopropyle ; $R_2$ est un hydrogène ; $R_3$ est un méthyle ; $R_4$ est un méthyle.

3. Procédé pour combattre les insectes des végétaux, de façon locale ou systémique, et pour protéger les plants cultivées, les arbres, les arbustes, le grain stocké et les plantes ornementales, ledit procédé étant caractérisé par l'application d'une quantité efficace en tant qu'insecticide du composé représenté par la formule développée (I), comme définie dans la revendication 1, et leurs sels acceptables aux points de vue pharmacologique et pharmaceutique.

4. Procédé selon la revendication 3, dans lequel ledit composé est appliqué au feuillage des cultures et plantes, sur le sol de culture ou sur leur tronc.

5. Procédé selon la revendication 4, dans lequel ledit composé comprend $R_1$ qui est un isopropyle ; $R_2$ un hydrogène ou un méthyle ; $R_3$ un méthyle ; $R_4$ un méthyle ; et dans lequel ledit composé comprend $R_1$ qui est un isopropyle ; $R_2$ un hydrogène ; $R_3$ un méthyle ; $R_4$ un méthyle.

**6.** Procédé pour contrôler les nématodes des végétaux, ledit procédé étant caractérisé par l'application au feuillage des plantes, sur le sol de culture ou sur leur tronc d'une quantité efficace en tant que nématocide du composé représenté par la formule développée (I), comme définie dans la revendication 1, et leurs sels acceptables aux points de vue pharmacoloqique et pharmaceutique.

**7.** Procédé selon la revendication 6, dans lequel ledit composé comprend $R_1$ qui est un isopropyle ; $R_2$ un hydrogène ou un méthyle ; $R_3$ un méthyle et $R_4$ un méthyle ; et dans lequel ledit composé comprend $R_1$ qui est un isopropyle ; $R_2$ un hydrogène ; $R_3$ un méthyle et $R_4$ un méthyle.

**8.** Composition pour le traitement, la prévention ou le contrôle des infections parasitaires des animaux à sang chaud, ladite composition étant caractérisée par : une quantité efficace aux points de vue prophylactique, thérapeutique ou pharmaceutique du composé représenté par la formule développée (I), commme définie dans la revendication 1, ou leurs sels acceptables aux points de vue pharmacoloqique et pharmaceutique ; et un vecteur inerte.

**9.** Composition pour combattre les insectes des végétaux, ladite composition étant caractérisée par : une quantité efficace en tant qu'insecticide du composé représenté par la formule développée (I), comme définie dans la revendication 1, ou leurs sels acceptables aux points de vue pharmacoloqique et pharmaceutique ; et un vecteur inerte.